# EUROPÄISCHE PATENTANMELDUNG

(11) **EP 1 201 666 A2**
(43) Veröffentlichungstag der Anmeldung: **02.05.2002**
(21) Anmeldenummer: 02001063.3
(22) Anmeldetag: 25.02.1998
(51) Int. Cl.: C07D 493/04

(54) **Seitenkettenmodifizierte Epothilone**

(30) Priorität: 25.02.1997 DE 19707505
(62) Teilanmeldung aus: 98912388.0
(71) Anmelder: Gesellschaft für Biotechnologische Forschung mbH (GBF), 38124 Braunschweig (DE)
(72) Erfinder: Höfle, Gerhard, Prof. Dr., 38124 Braunschweig (DE); Sefkow, Michael, 14469 Potsdam (DE); Gerth, Klaus, Dr., 38124 Braunschweig (DE); Steinmetz, Heinrich, 38124 Braunschweig (DE)
(74) Vertreter: Boeters, Hans Dietrich, Dr.

(57) **Zusammenfassung**

Die Erfindung betrifft seitenkettenmodifizierte Epothilone.

## Beschreibung

Epothilone A und B sind bekannt; vgl. beispielsweise DE 4 138 042, WO 93 10 121 und WO 97 19 086. Nach WO 97 19 086 können die Epothilone A und B in 3- und/oder 7-Stellung beispielsweise durch C₁₋₆-Alkyl, C₁₋₆-Acyl, Benzoyl, C₁₋₄-Trialkylsilyl, Benzyl, Phenyl, C₁₋₆-Alkoxy oder hydroxy- oder halogensubstituiertes Benzyl oder Phenyl geschützt sein.

Der genannte Stand der Technik schlägt sie für therapeutische Mittel vor. In PNAS USA, 95 (1998) 1369 - 1374 werden Epothilone als nützliche therapeutische Mittel bezeichnet. Infolge ihrer therapeutischen Effekte werden nach Angew. Chem. Int. Ed., 36 (1997) 2097 - 2103 sogar eine umfangreiche Bibliothek derartiger Verbindungen (extensive library of compounds) vorgesehen.

Die Erfindung betrifft nun ein Verfahren zur Herstellung von in 16,17-Stellung modifizierten Epothilonen, bei dem man von 3,7-geschützten oder ungeschützten Epothilonen A oder B ausgeht und
a) diese an der 16,17-Doppelbindung hydriert oder
b) an der 16,17-Doppelbindung Halogen addiert oder
c) an der 16,17-Doppelbindung epoxidiert und gegebenenfalls das erhaltene Epoxid zum 16-Alkohol reduziert.

Das erfindungsgemäße Verfahren kann dadurch gekennzeichnet sein, daß man bei
- Methode (a) mit Diimin oder Wasserstoff und einem heterogenen oder homogenen Metallkatalysator hydriert oder bei
- Methode (c) mit einer Persäure oder einem Dioxiran epoxidiert.

Ferner kann dieses erfindungsgemäße Verfahren dadurch gekennzeichnet sein, daß man die Katada-Reaktion mit Acetanhydrid durchführt und die gewonnenen 21-Acetoxyepothilone gegebenenfalls in an sich bekannter Weise zu 21-Hydroxy-epothilonen A oder B spaltet (Epothilone E bzw. F).

Ferner kann dieses erfindungsgemäße Verfahren dadurch gekennzeichnet sein, daß man die fakultative Spaltung hydrolytisch oder enzymatisch durchführt.

Ferner betrifft die Erfindung ein Verfahren zur Herstellung von in C19-Stellung modifizierten Epothilonen, bei dem man 3,7-geschützte oder ungeschützte Epothilone A oder B in C19-Stellung metalliert und in an sich bekannter Weise mit elektrophilen Reagenzien als in C19-Stellung modifizierte alkyl-, aryl-, heteroaryl-, halogen-, sauerstoff- oder schwefelsubstituierte Epothilone abfängt.

Dieses erfindungsgemäße Verfahren kann dadurch gekennzeichnet sein, daß man mit Butyllithium metalliert.

Ferner betrifft die Erfindung ein Verfahren zur Herstellung von in C27-Stellung modifizierten Epothilonen, bei dem man die Allylgruppierung (C17, C16 und C27) in an sich bekannter Weise an der C27-Methylgruppe durch ein Heteroatom substituiert.

Dieses erfindungsgemäße Verfahren kann dadurch gekennzeichnet sein, daß man die C27-Methylgruppe mit einem Bromatom substituiert, insbesondere mit Hilfe von N-Bromsuccinimid, und das erhaltene Bromid gegebenenfalls in eine C27-Hydroxy-Verbindung überführt.

Schließlich betrifft die Erfindung nach dem erfindungsgemäßen Verfahren hergestellte Verbindungen.

Ferner betrifft die Erfindung ein Verfahren zur Herstellung von 2,3-ungesättigten Epothilon-N-oxiden, bei dem man entweder
(i) 3,7-geschützte Epothilone A oder B in an sich bekannter Weise in ein N-Oxid überführt und basisch den 3-Substituenten zur 2,3-Doppelbindung eliminiert oder
(ii) 7-geschützte oder 7-ungeschützte Epothilone A oder B, die in 2,3-Stellung eine Doppelbindung aufweisen, auf an sich bekannte Weise in ein N-Oxid überführt und
gegebenenfalls das erhaltene N-Oxid einer O-Alkylierung unterwirft und ein O-Alkylierungsprodukt gewinnt.

Ferner betrifft die Erfindung ein Verfahren zur Herstellung von Epothilon-N-oxiden, bei dem man 3,7-geschützte oder ungeschützte Epothilone A oder B in an sich bekannter Weise in ein N-Oxid überführt und das erhaltene N-Oxid gegebenenfalls einer O-Alkylierung unterwirft und ein O-Alkylierungsprodukt gewinnt.

Dieses erfindungsgemäße Verfahren kann dadurch gekennzeichnet sein, daß man die N-Oxidierung mit Persäure oder einem Dioxiran durchführt und für die fakultative O-Alkylierung elektrophile Alkyl-, Aryl- oder Heteroaryl-Reagenzien verwendet, insbesondere Methyliodid oder Trimethyloxoniumtetrafluorborat.

Ferner kann dieses erfindungsgemäße Verfahren dadurch gekennzeichnet sein, daß man ein erhaltenes N-Oxid einer Katada-Reaktion unterwirft, insbesondere gemäß Houben-Weyl, Band E7b, Seite 646.

Ferner kann dieses erfindungsgemäße Verfahren dadurch gekennzeichnet sein, daß man die Katada-Reaktion mit einem aktivierten Carbonsäurederivat durchführt, insbesondere Carbonsäureanhydrid oder Carbonsäurechlorid.

### Versuch 1: Diepoxyepothilon A. (1a)

Eine Lösung von Epothilon A (5 mg, 10 µmol) in Aceton (1 ml) wurde bei 0 °C mit Dimethyldioxiran (0.4 ml, 28 µmol, 0.07 M in Aceton) versetzt. Die Lösung wurde über einige Stunden auf Raumtemperatur gebracht und 20 h bei dieser Temperatur gerührt. Da nach DC noch Edukt vorhanden war, wurde weiteres Dimethyldioxiran (0.25 ml, 17 µmol) zugegeben und das Reaktionsgemisch erneut 20 h bei Raumtemperatur gerührt. Das Lösungsmittel wurde entfernt und der Rückstand mittels PSC (0.25 x 200 x 200 mm, 10% MeOH:CH₂Cl₂) gereinigt. Es wurden isoliert:
1. 1.4 mg (27%) Diepoxyepothilon A (3:2 Epimerengemisch an C16-C17). R_{*f*}: 0.63 (10% MeOH:CH₂Cl₂); R_{*t*}: 6.79 (Isomer 1) und 7.39 (Isomer 2) min (RP 18, 250 x 4 mm, MeOH:H₂O 65:35, 1 ml/min); MS: (m/z) = 510 (M⁺); ¹H-NMR (400 MHz, CDCl₃, ausgewählte Signale, Isomer 1): δ = 6.96 (s, 1H, H-19), 5.48 (dd, *J*= 12.2 und 2.5 Hz, 1H, H-15), 4.37 (dbr, *J* = 10.7 Hz, 1H, H-3), 4.10 (s, 1H, H-17), 3.67 (dd, *J* = 5.6 und 2.5 Hz, 1H, H-7), 3.14 (qd, *J*= 6.6 und 2.5 Hz, 1H, H-6), 3.00 (ddd, *J*= 9.7, 3.6 und 2.5 Hz, 1H, H-13), 2.88 (dt, *J* = 8.6 und 3.6 Hz, 1H, H-12), 2.71 (s, 3H, H-21), 2.53 (dd, *J* = 13.7 und 11.7 Hz, 1H, H-2a), 1.41 (s, 3H, H-22), 1.27 (s, 3H, H-26), 1.17 (d, *J* = 6.6 Hz, 3H, H-24), 1.08 (s, 3H, H-23), 0.97 (d, *J* = 7.1 Hz, 3H, H-25); (Isomer 2) δ = 6.98 (s, 1H, H-19), 5.11 (dd, *J* = 11.7 und 2.5 Hz, 1H, H-15), 4.27 (dbr, *J* = 10.7 Hz, 1H, H-3), 4.14 (s, 1H, H-17), 3.06 (qd, *J* = 6.6 und 2.9 Hz, 1H, H-6), 2.96 (ddd, *J* = 9.7, 3.6 und 2.5 Hz, 1H, H-13), 2.31 (dt, *J* = 14.7 und 2.0 Hz, 1H, H-14a), 1.36 (s, 3H, H-22), 1.15 (d, *J* = 6.6 Hz, 3H, H-24), 1.14 (s, 3H, H-26), 1.07 (s, 3H, H-23).
2. 0.8 mg (16%) Epothilon A N-Oxid. R_{*f*}: 0.44 (10% MeOH:CH₂Cl₂); R_{*t*}: 4.25 min (RP 18, 250 x 4 mm, MeOH:H₂O 65:35, 1 ml/min); MS: (m/z) = 510 (M⁺); ¹H-NMR: siehe Methode 1

### Versuch 2: Dihydroepothilon A. (1c)

Zu einer Lösung von Epothilon A (11 mg, 22 µmol) in Ethanol (2 ml) wurde Palladium auf Aktivkohle (5 mg, 10%) gegeben und die schwarze Suspension 24 h bei Raumtemperatur einer H₂-Amosphäre ausgesetzt. Da die Umsetzung nach DC noch nicht vollständig war, wurde eine weitere Portion Pd/C zugesetzt und das Reaktionsgemisch weitere 20 h unter einer H₂-Atmosphäre gerührt. Die Trennung der Produkte erfolgte mittels PSC (1 x 200 x 200 mm, 10% MeOH:CH₂Cl₂). Es wurden isoliert:
1. 0.5 mg (5%) Dihydroepothilon A. R_{*f*}: 0.60 (10% MeOH:CH₂Cl₂); R_{*t*}: 10.80 min (RP 18, 250 x 4 mm, MeOH:H₂O 65:35, 1 ml/min); MS: (m/z) = 496 (M⁺), 478, 408, 308; ¹H-NMR (400 MHz, CDCl₃, ausgewählte Signale): δ = 7.05 (d, *J* = 6.6 Hz, 1H, OH), 6.77 (s, 1H, H-19), 5.23 (dd, *J* = 12.4 und 2.3 Hz, 1H, H-15), 4.42 (ddd, *J* = 11.7, 6.6 und 3.0 Hz, 1H, H-3), 3.70 (ddd, *J* = 5, 3 und 2 Hz, 1H, H-7), 3.12 (qd, *J* = 6.6 und 3.0 Hz, 1H, H-6), 3.07 (d, *J* = 12.7 Hz, 1H, H-17a), 2.96 (ddd, *J* = 9.7, 3.6 und 2.0 Hz, 1H, H-13), 2.91 (ddd, *J* = 9.7, 3.6 und 2.6 Hz, 1H, H-12), 2.68 (s, 3H, H-21), 2.51 (dd, *J* = 13.7 und 11.7 Hz, 1H, H-2a), 2.24 (d, *J* = 12.7 Hz, 1H, H-17b), 2.19 (m, 1H, H-16), 2.13 (dd, *J* = 13.7 und 3.0 Hz, 1H, H-2b), 1.35 (s, 3H, H-22), 1.15 (d, *J* = 6.6 Hz, 3H, H-24), 1.09 (s, 3H, H-23), 0.99 (d, *J* = 7.1 Hz, 3H, H-25), 0.93 (d, *J* = 6.6 Hz, 3H, H-26).
2. 8 mg (72%) 15-Deoxy-dihydroepothilonsäure. R_{*f*}: 0.10 (10% MeOH:CH₂Cl₂).

### Versuch 3: 16-Hydroxyepothilon A. (1b)

Zu einer Lösung von Diepoxyepothilon A (7 mg, 14 µmol), 1:1 Epimerengemisch an C-16) in Ethanol (2 ml) wurde Palladium auf Aktivkohle (10 mg, 10%) gegeben und die schwarze Suspension 24 h bei Raumtemperatur einer H₂-Amosphäre ausgesetzt. Da die Umsetzung nach . DC noch nicht vollständig war, wurde eine weitere Portion Pd/C zugesetzt und das Reaktionsgemisch weitere 80 h unter einer H₂-Atmosphäre gerührt. Die Trennung der Produkte erfolgte mittels PSC (1 x 200 x 200 mm, 10% MeOH:CH₂Cl₂). Es wurden isoliert:
1. 3 mg (43%) 16-Hydroxyepothilon A (Isomer 1). R_{*f*}: 0.38 (10% MeOH:CH₂Cl₂); R_{*t*}: 6.65 min (RP 18, 250 x 4 mm, MeOH:H₂O 65:35, 1 ml/min); ¹H-NMR (400 MHz, CDCl₃, ausgewählte Signale): δ = 6.85 (s, 1H, H-19), 5.02 (dd, *J* = 11.7 und 2.0 Hz, 1H, H-15), 4.38 (dbr, *J*= 11.2 Hz, 1H, H-3), 3.67 (dd, *J*= 4 und 3 Hz, 1H, H-7), 3.14 (qd, *J* = 6.8 und 3.0 Hz, 1H, H-6), 2.95 (d, *J* = 15.3 Hz, 1H, H-17a), 2.89 (d, *J* = 15.3 Hz, 1H, H-17b), 2.89 (ddd, *J* = 10.2, 3.6 und 2.0 Hz, 1H, H-13), 2.81 (ddd, *J* = 9.7, 3.6 und 2.5 Hz, 1H, H-12), 2.70 (s, 3H, H-21), 2.53 (dd, *J*= 15.8 und 11.7 Hz, 1H, H-2a), 2.14 (dd, *J* = 15.8 und 2.0 Hz, 1H, H-2b), 2.08 (dt, *J* = 14.3 und 2.0 Hz, 1H, H-14a), 1.39 (s, 3H, H-22), 1.25 (s, 3H, H-26), 1.19 (d, *J* = 6.6 Hz, 3H, H-24), 1.05 (s, 3H, H-23), 0.99 (d, *J* = 7.1 Hz, 3H, H-25).
2. 3 mg (43%) 16-Hydroxyepothilon A (Isomer 2). R_{*f*}: 0.31 (10% MeOH:CH₂Cl₂); R_{*t*}: 6.10 min (RP 18, 250 x 4 mm, MeOH:H₂O 65:35, 1 ml/min); ¹H-NMR (300 MHz, CDCl₃, ausgewählte Signale): δ = 6.85 (s, 1H, H-19), 5.21 (dd, *J* = 11.3 und 1.9 Hz, 1H, H-15), 4.42 (dbr, *J*= 10.5 Hz, 1H, H-3), 3.71 (sbr, 1H, H-7), 3.21 (d, *J* = 14.3 Hz, 1H, H-17a), 3.13 (qd, *J* = 6.8 und 3.0 Hz, 1H, H-6), 3.09 (dt, *J* = 9.8 und 3.4 Hz, 1H, H-13), 2.87 (dt, *J* = 9.4 und 3.0 Hz, 1H, H-12), 2.73 (d, *J* = 14.3 Hz, 1H, H-17b), 2.68 (s, 3H, H-21), 2.63 (dd, *J =* 16.6 und 11.7 Hz, 1H, H-2a), 2.27 (dt, *J*= 14.7 und 2.3 Hz, 1H, H-14a), 2.24 (dd, *J* = 16.6 und 2.6 Hz, 1H, H-2b), 1.39 (s, 3H, H-22), 1.22 (s, 3H, H-26), 1.19 (d, *J* = 6.8 Hz, 3H, H-24), 1.05 (s, 3H, H-23), 0.99 (d, *J* = 7.2 Hz, 3H, H-25).

**Epothilon A-*N*-oxid ( 2a ):** Zu 100 mg Epothilon A in 1 ml Dichlormethan werden 100 mg 70%ige *m*-Chlorperbenzoesäure in 0.5 ml Dichlormethan gegeben. Nach 6-stündigem Rühren bei Raumtemperatur wird mit Dichlormethan verdünnt und nacheinander mit Natruiumsulfitlösung zur Zerstörung von überschüssiger Persäure und Natriumbicarbonatlösung ausgeschüttelt. Das Lösungsmittel wird i. Vak abgedampft, der Rückstand durch präparative HPLC an einer Nucleosil RP-18 Säule ( 250 x 20 mm, Laufmittel Methanol/Wasser 60 :40 ) aufgetrennt. Ausbeute 60 mg farbloses Öl.
R_{f}- 0.60 ( Kieselgel DC Alufolie, Laufmittel Dichlormethan/Methanol 9:1);
ESI-MS (neg. Ionen) m/z 510;
UV (Methanol); lamda max. 240nm;
¹³C-NMR (CDCl₃): C-1 70,5, C-2 39,9, C-3 70,8, C-4 55.1, C-5 221.4, C-6 40.9, C-7 72.9, C-8 37.6, C-9 31.8, C-10 22.8, C-11 28.0, C-12 58.0, C-13 55.8, C-14 32.2, C-15 75.5, C-16 144,5, C-17 111.4, C-18 143.4, C-19 110.3, C-20 145.6, C-21 13.5, C-22 15.4, C-23 23.3, C-24 12.0, C-25 16.5, C-27 18.2 ppm;

**21-Acetoxyepothilon A (= 21-Acetylepothilon E) (3a):** Zu 50 mg Epothilon A-*N*-oxid (2a) in 0.5 ml Dichlormethan werden 0.05 ml 2,6-Di-tert.-butylpyridin und 0.1 ml Acetanhydrid gegeben. Nach 15 Minuten Erwärmen auf 75°C werden im Vakuum Lösungsmittel und Reagenzien abgedampft, der Rückstand durch präparative HPLC an Nucleosil RP-18 ( 250 x 20 mm, Laufmittel Methanol / Wasser 60 : 40) aufgetrennt. Ausbeute 30 mg farbloses Öl.
R_{f}- 0.50 (Kieselgel DC Alufolie, Laufmittel Dichlormethan/Methanol 95:5);
ESI-MS (neg. Ionen): m/z 552;
UV (Methanol) lamda max. 210, 250 nm;
¹H-NMR (CDCl₃, gegenüber **2a** veränderte Signale): 15-H 5.45 dd, 17-H 6.60 s, 19-H 7.15 s, 21-H₂ 5.35 s, CH₃CO 2.15 s ppm.

**Epothilon E ( 3b ):** Zu 10 mg 21-Acetoxyepothilon A (3a) in 0.5 ml Methanol gibt man 1 Tropfen konz. Ammoniaklösung, erwärmt 1 Stunde auf 40°C und dampft i. Vak. zur Trockene ein. Der Rückstand wird durch präparative DC aufgetrennt. Ausbeute 6mg, identisch mit einer authentischen Probe Epothilon E.

### Versuch 4: 19-Methylepothilon A. (4b)

Eine Lösung von Epothilon A (15 mg, 30 µmol) in THF (1 ml) wurde bei -90 °C mit *n*-Butyllithium (100 µl, 160 µmol, 1.6 M in Hexan) versetzt. Die Lösung färbte sich sofort goldorange. Nach 15 min Rühren bei -90 °C wurde die Reaktionslösung mit Methyliodid (100 µl, 1.6 mmol) versetzt. Die resultierende schwach grünlichgelbe Lösung wurde auf -30 °C erwärmt und mit pH = 7.0 Puffer (2 ml) gequenscht. Mit 0.1 N Salzsäure wurde die Emulsion auf pH 6 gebracht. Nach Sättigung mit festem NaCl wurde die wäßrige Phase mit CH₂Cl₂ (2 x 5 ml) und Ethylacetat (5 ml) extrahiert, die vereinigten organischen Phasen über MgSO₄ getrocknet, filtriert und das Lösungsmittel am Rotavap entfernt. Die Reinigung erfolgte über PSC (1 x 200 x 200 mm, 10% MeOH:CH₂Cl₂) und HPLC (RP 18, 250 x 16 mm, MeOH:H₂O 65:35). Es wurden isoliert:
1. 2.5 mg (17%) 19-Methylepothilon A. R_{*f*}: 0.50 (10% MeOH:CH₂Cl₂); R_{*t*}: 11.70 min (RP 18, 250 x 4 mm, MeOH:H₂O 65:35, 1 ml/min); MS: (m/z) = 508 (M⁺), 420, 320; ¹H-NMR (300 MHz, CDCl₃, ausgewählte Signale): δ = 6.41 (s, 1H, H-17), 5.46 (dd, *J* = 9.0 und 2.3 Hz, 1H, H-15), 4.15 (dd, *J* = 10.5 und 3.0 Hz, 1H, H-3), 3.77 (dd, *J*= 8 und 4 Hz, 1H, H-7), 3.20 (qd, *J*= 6.8 und 4.5 Hz, 1H, H-6), 3.04 (dt, *J* = 7.5 und 3.8 Hz, 1H, H-13), 2.91 (dt, *J* = 7.5 und 3.8 Hz, 1H, H-12), 2.61 (s, 3H, H-21), 2.51 (dd, *J* = 14.4 und 10.5 Hz, 1H, H-2a), 2.38 (dd, *J* = 14.4 und 3.0 Hz, 1H, H-2b), 2.32 (s, 3H, H-27), 2.15 (ddd, *J* = 15.1, 3.8 und 3.0 Hz, 1H, H-14a), 2.01 (d, *J* = 1.5 Hz, 3H, H-26), 1.91 (dt, *J* = 15.1 und 8.8 Hz, 1H, H-14b), 1.34 (s, 3H, H-22), 1.16 (d, *J* = 6.8 Hz, 3H, H-24), 1.10 (s, 3H, H-23), 1.00 (d, *J*= 6.8 Hz, 3H, H-25).
2. ca. 50% Epothilon A

### Versuch 5: 19-Bromepothilon A. (4a)

Eine Lösung von Epothilon A (25 mg, 50 µmol) in THF (2.5 ml) wurde bei -90 °C mit *n*-Butyllithium (160 µl, 225 µmol, 1.6 M in Hexan) versetzt. Die Lösung färbte sich sofort goldorange. Nach 15 min Rühren bei -90 °C wurde N-Bromsuccinimid (27 mg, 150 µmol), gelöst in THF (0.5 ml), hinzugegeben. Die Lösung entfärbte sich langsam. Die nun schwch bräunliche Reaktionsmischung wurde auf -30 °C erwärmt und mit 0.1 N Salzsäure (1 ml) auf pH 6.5 gebracht. Nach Sättigung mit festem NaCl wurde die wäßrige Phase mit CH₂Cl₂ (2 x 5 ml) und Ethylacetat (5 ml) extrahiert, die vereinigten organischen Phasen über MgSO₄ getrocknet, filtriert und das Lösungsmittel am Rotavap entfernt. Die Reinigung erfolgte über PSC (1 x 200 x 200 mm, 10% MeOH:CH₂Cl₂) und HPLC (RP 18, 250 x 16 mm, MeOH:H₂O 65:35). Es wurden isoliert:
1. 2.6 mg (9%) 19-Bromepothilon A. R_{*f*}: 0.53 (10% MeOH:CH₂Cl₂); R_{*t*}: 20.78 min (RP 18, 250 x 4 mm, MeOH:H₂O 65:35, 1 ml/min); MS: (m/z) = 574 und 572 (M⁺), 556, 554, 468, 466, 386, 384, 341; ¹H-NMR (300 MHz, CDCl₃, ausgewählte Signale): δ = 6.43 (s, 1H, H-17), 5.46 (dd, *J*= 8.7 und 2.3 Hz, 1H, H-15), 4.13 (ddd, *J*= 9.4, 6.0 und 3.8 Hz, 1H, H-3), 3.80 (dd, *J*= 8 und 4 Hz, 1H, H-7), 3.38 (d, *J*= 6.0 Hz, 1H, OH), 3.22 (qd, *J*= 6.8 und 5.3 Hz, 1H, H-6), 3.05 (dt, *J*= 8.3 und 4.1 Hz, 1H, H-13), 2.91 (dt, *J*= 7.5 und 3.7 Hz, 1H, H-12), 2.66 (s, 3H, H-21), 2.55 (dd, *J*= 14.7 und 9.4 Hz, 1H, H-2a), 2.47 (dd, *J* = 14.7 und 3.8 Hz, 1H, H-2b), 2.16 (d, *J*= 1.1 Hz, 3H, H-26), 2.14 (dt, *J* = 14.7 und 3.8 Hz, 1H, H-14a), 1.90 (dt, *J*= 15 und 8.3 Hz, 1H, H-14b), 1.34 (s, 3H, H-22), 1.17 (d, *J* = 6.8 Hz, 3H, H-24), 1.11 (s, 3H, H-23), 1.01 (d, *J*= 6.8 Hz, 3H, H-25).
2. ca. 60% Epothilon A.

### Synthesebeispiele 1a bis 5a

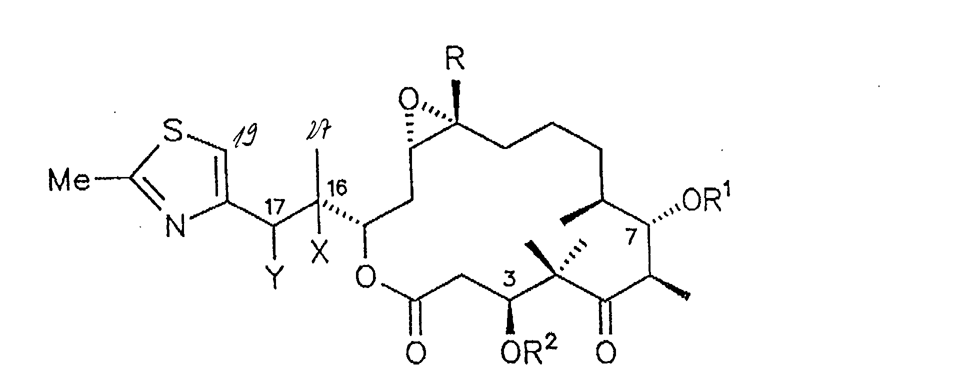

**1a** R¹, R² = H, X,Y = -O-, R = H

**b** R¹, R² H, X = OH Y = H , R = H

**c** R¹, R² = H, X = H Y = H, R = H

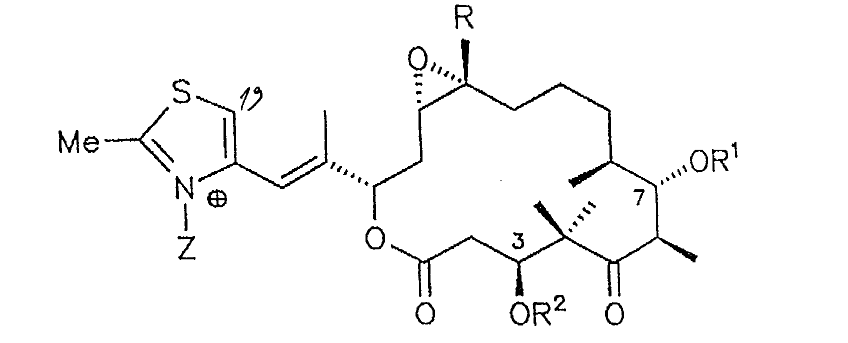

**2a** R¹, R² = H, Z = O⁻, R = H

**b** R¹, R² = H, Z = OCH₃ BF₄⁻, R = H

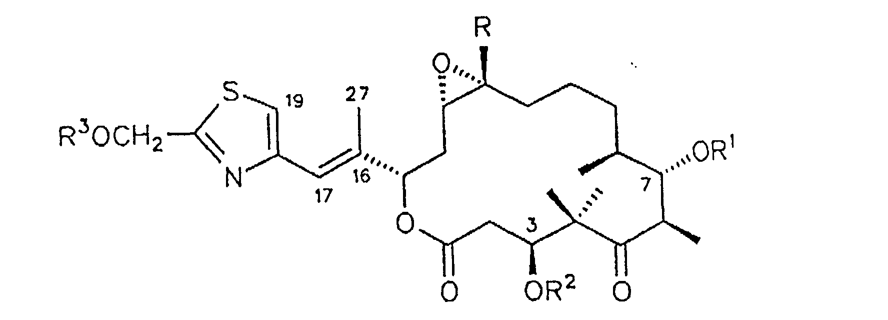

**3a** R¹, R² = H, R³ = Acetyl, R = H

**b** R¹, R², R³ = H, R = H

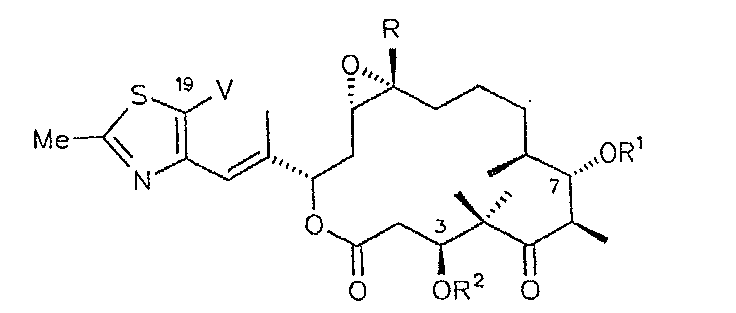

**4a** R¹, R² = H, V = Br, R = H

**b** V = CH₃, R¹, R² H, R = H

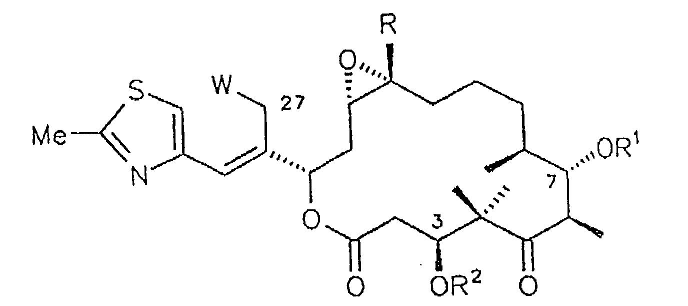

5a R¹, R² = H, W = OH, R = H

## Patentansprüche

1. Verfahren zur Herstellung von Epothilon-N-oxiden, bei dem man 3,7-geschützte Epothilone A oder B in an sich bekannter Weise in ein N-Oxid überführt.

2. Verfahren nach Anspruch 1, **dadurch gekennzeichnet, daß** man die N-Oxidierung mit Persäure oder einem Dioxiran durchführt.

3. N-Oxid von 3,7-geschütztem Epothilon A.

4. N-Oxid von 3,7-geschütztem Epothilon B.
